(19) Europäisches Patentamt
European Patent Office
Office européen des brevets

(11) Veröffentlichungsnummer: **0 161 629**
**B1**

(12) **EUROPÄISCHE PATENTSCHRIFT**

(45) Veröffentlichungstag der Patentschrift: **03.08.88**

(21) Anmeldenummer: **85105610.1**

(22) Anmeldetag: **08.05.85**

(51) Int. Cl.[4]: **C 07 K 7/10**, C 07 K 7/08, C 12 N 15/00, C 12 P 19/34, C 12 P 21/00, C 12 N 1/20 // C12R1/465

(54) **Signalpeptid für die Exkretion von Peptiden in Streptomyceten.**

(30) Priorität: **17.05.84 DE 3418274**

(43) Veröffentlichungstag der Anmeldung: **21.11.85 Patentblatt 85/47**

(45) Bekanntmachung des Hinweises auf die Patenterteilung: **03.08.88 Patentblatt 88/31**

(84) Benannte Vertragsstaaten: **AT BE CH DE FR GB IT LI LU NL SE**

(56) Entgegenhaltungen: **DE - A - 3 331 860**

(73) Patentinhaber: **HOECHST AKTIENGESELLSCHAFT, Postfach 80 03 20, D-6230 Frankfurt am Main 80 (DE)**

(72) Erfinder: **Koller, Klaus-Peter, Dr., Am Flachsland 18, D-6233 Kelkheim (Taunus) (DE)**

**EP 0 161 629 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die nachstehend definierte Erfindung betrifft ein Signalpeptid, das Bestandteil eines Propeptids ist, welches in einer Streptomyceten-Zelle, die eine Signalpeptidase enthält, in das Signalpeptid und ein Polypeptid gespalten wird, wobei letzteres aus der Zelle entfernt und in das Kulturmedium ausgeschieden wird. Die Erfindung betrifft weiterhin DNA-Sequenzen, die für dieses Signalpeptid codieren, Genstrukturen, die diese DNA-Sequenz im Leserahmen mit einem Strukturgen enthalten, Plasmide, die eine solche Genstruktur enthalten, sowie Wirtsorganismen mit solchen Plasmiden. Weitere Aspekte der Erfindung sowie deren bevorzugte Ausgestaltungen sind im folgenden näher erläutert.

In der DE-A-33 31 860 wurde bereits ein Verfahren zur Herstellung von Tendamistat durch Fermentation von Streptomyces tendae vorgeschlagen, das dadurch gekennzeichnet ist, dass Tendamistat produzierende S. tendae-Stämme eingesetzt werden, die mit sublethalen Dosen Acriflavin behandelt wurden. Aus so erhaltenen Stämmen wurde ein DNA-Fragment mit dem Gen für Tendamistat isoliert, und zwar ein 2,3 kb-Pst I-Fragment. Durch Einbau dieses Fragments in mit Pst I geschnittenem pBR 322 konnte diese DNA in E. coli amplifiziert und wieder isoliert werden.

Es wurde nun gefunden, dass auf diesem 2,3 kb-Fragment unmittelbar vor dem Strukturgen für Tendamistat das Signalpeptid (Präpeptid) der Formel I

Met–Arg–Val–Arg–Ala–Leu–Arg–X–Ala–Ser–Ala (I)

codiert ist, wobei X für einen hydrophoben Bereich steht, der 10 bis 25, vorzugsweise 17 bis 20 Aminosäuren umfasst.

Es wurde weiterhin gefunden, dass dieses Signalpeptid befähigt ist, auch andere Peptide aus Wirtszellen auszuschleusen, die eine entsprechende Signalpeptidase enthalten. Die Erfindung betrifft deshalb auch Propeptide der Formel II

Sig–R (II)

in der Sig die Aminosäuresequenz der Formel I bedeutet und R für den Rest eines aminoterminal verknüpften genetisch codierbaren Peptids steht, bei dem vorzugsweise am Amino-Terminus eine saure Aminosäure, insbesondere Asparaginsäure, steht.

Ein Aspekt der Erfindung betrifft somit Peptide der Formel II, in der R Wasserstoff oder einen Peptidrest, beispielsweise den Tendamistatrest, bedeutet. Ein anderer Aspekt der Erfindung betrifft die entsprechende DNA-Sequenz, die erhältlich ist aus Tendamistat-produzierenden Streptomyces tendae-Stämmen, die mit sublethalen Dosen Acriflavin behandelt wurden, durch Isolieren der Gesamt-DNA, Verdauen mit Pst I, Southern-Hybridisierung mit der DNA-Sequenz A

5'-($^{32}$P-)CCT TCA GTG TCG TCT TCG TA-3' (A)

Isolieren des 2,3 kb-Pst I-Fragments, Schneiden mit BamHI, Southern-Hybridisieren mit der Sequenz A, Isolieren des 0,94 kb-PstI-BamHI-Subfragments, Schneiden mit Sau 3a, Southern-Hybridisieren mit der Sequenz A, Isolieren des 0,525 kb-BamHI-Sau 3a-Subfragments und Sequenzieren der DNA und die gekennzeichnet ist durch die Merkmale:

a) sie liegt unmittelbar vor dem Tendamistat-Strukturgen,

b) sie codiert am Amino-Terminus für Met-Arg-Val-Arg-Ala-Leu-Arg,

c) sie codiert am Carboxy-Terminus für Ala-Ser-Ala und

d) sie codiert in der Mitte für einen hydrophoben Bereich X, der 10 bis 25, vorzugsweise 17 bis 20 Aminosäuren umfasst.

Diese für das Signalpeptid codierende DNA-Sequenz wird im folgenden als Sequenz B bezeichnet.

Die durch Vergleich mit Standardmarkern ermittelten kb-Zahlenwerte haben die übliche Genauigkeit.

Anstelle der Sequenz A kann zur Southern-Hybridisierung eine beliebige, dem Tendamistatgen oder dem Gegenstrang komplementäre Sequenz gewählt werden.

Für die verschiedenen Schritte zur Charakterisierung der DNA-Sequenz B wird in der Praxis jeweils die DNA in einen geeigneten Vector eingebracht, dieser in eine Wirtszelle transformiert, dort amplifiziert, durch Kolonie-Hybridisierung mit der Sequenz A die Transformanten ermittelt und die DNA reisoliert. Diese Schritte sind an sich bekannt.

Die DNA-Sequenz C, deren codierender Strang im Anhang niedergelegt ist, zeigt die Nucleotidfolge des Tendamistat Strukturgens aus S. tendae.

Die genannten Genstrukturen enthalten somit die DNA-Sequenz B im Leserahmen mit einem Strukturgen, welches beispielsweise für Tendamistat codiert, und zwar vorzugsweise die DNA-Sequenz C.

Die Erfindung betrifft weiterhin Plasmide, die gekennzeichnet sind durch die DNA-Sequenz B, im Leserahmen mit einem Strukturgen, welches beispielsweise für Tendamistat codiert, insbesondere für die DNA-Sequenz C. Diese Plasmide können ein in E. coli wirksames Replicon enthalten und sind dann befähigt, die DNA in E. coli zu amplifizieren und gegebenenfalls auch zu exprimieren.

Bevorzugte Plasmide enthalten zusätzlich ein in Streptomyceten wirksames Replicon. Wird mit einem solchen Plasmid ein Streptomycet transformiert, so wird er befähigt, das durch das Strukturgen determinierte Peptid in Form des Propeptids der Formel II zu exprimieren, welches dann im Rahmen der Prozessierung durch eine Signalpeptidase gespalten und das gewünschte Peptid in das Kulturmedium exkretiert wird.

Vorteilhaft sind auch sogenannte «Shuttle»-Plasmide, die sowohl ein in E. coli als auch ein in Streptomyceten wirksames Replicon enthalten. Diese «Shuttle»-Vectoren können in E. coli amplifiziert und nach Reisolierung in Streptomyceten transformiert werden, wo dann die Produktion des gewünschten Polypeptids erfolgt.

Die Erfindung betrifft auch Wirtsorganismen, die mit den genannten Plasmiden transformiert wurden, insbesondere Wirtsorganismen der Gattung Streptomyces, vor allem der Art S. tendae oder insbesondere S. lividans.

Die Erfindung betrifft weiterhin ein Verfahren zur Herstellung eines Polypeptids der allgemeinen Formel III

$H_2N-R$ (III)

in der R die bei Formel II genannte Bedeutung hat, bei dem man einen der genannten transformierten Wirtsorganismen einsetzt, welcher eine Signalpeptidase enthält, die das Propeptid der Formel II spaltet und das gewünschte Polypeptid sekretiert.

Während es für gram-negative Bakterien eine Fülle von Vectoren gibt, sind für gram-positive Bakterien nur wenige Vectoren beschrieben. Vectoren für Bakterien der Art. S. tendae sind bisher nicht bekannt. Die Erfindung ermöglicht somit einen Zugang für die Nutzung von S. tendae als Wirtsorganismen.

Ein besonderer Vorteil der Erfindung liegt darin, dass transformierte Streptomyces-Stämme, insbesondere S. lividans-Stämme, optimal versporen, das heisst, der Gehalt an dem rekombinanten Plasmid beeinträchtigt diese Stämme in ihrer generativen Phase nicht. Die transformierten Organismen sind somit auch für weitere Stammverbesserungen geeignet, beispielsweise zur Herstellung und Selektion von metabolischen Mutanten, bei deren Herstellung mit Sporen gearbeitet wird.

Gegenüber den bekannten Stämmen von S. tendae bilden die transformierten Stämme, insbesondere S. lividans, kein Melanin. Es entfällt somit dessen Abtrennung, was die Isolierung des erwünschten Peptids, beispielsweise Tendamistat, wesentlich erleichtert und Ausbeuteverluste verhindert.

Ein weiterer Vorzug der Erfindung liegt darin, dass Fremdgene auch in S. lividans exprimiert werden und die entsprechenden Polypeptide exkretiert werden, was ebenfalls vielfältige Möglichkeiten zur Stammverbesserung und auch Modifizierung des so hergestellten Polypeptids bietet.

Erfindungsgemäss können jedoch auch andere Streptomyces-Arten transformiert werden, beispielsweise S. ghanaensis oder aureofaciens. Werden plasmidfreie Stämme, die in der Lage sind, eine spezifische Signalpeptidase zu synthetisieren, mit den erfindungsgemässen Hybridplasmiden transformiert, so werden stabile Transformanten erhalten, die das codierte Peptid exprimieren und sekretieren.

Besonders bevorzugte Ausgestaltungen der Erfindung sind in den folgenden Beispielen näher erläutert. Prozentangaben beziehen sich hierbei auf das Gewicht, sofern nichts anderes vermerkt ist. Die Hybridplasmide darstellenden Figuren zeigen die Schnittstellen massstabsgerecht.

In den Beispielen wurden die folgenden literaturbekannten Vectoren eingesetzt: Einzelstrang-Phagen M 13 mp 8 und M 13 mp 9: Messing et al., Gene 19 (1982) 269; pUC 8: Vierra et al., Gene 19 (1982) 259; pAC 177 und 184: Chang et al., J. Bacteriology 134 (1978) 1141; pIJ 102 und 350: Kieser et al., Mol. Gen. Genet. 185 (1982) 223.

Die Stammhaltung von S. tendae ist in der US-Patentschrift 4 226 764 beschrieben. Die Isolierung des Tendamistatgens kann grundsätzlich aus jedem Tendamistat produzierenden Stamm erfolgen. Besonders vorteilhaft ist jedoch das Vorgehen gemäss der deutschen Patentanmeldung P 33 31 860.3, in deren Beispiel 3 die Isolierung der DNA beschrieben ist. Diese isolierte Gesamt-DNA ist das Ausgangsmaterial für das folgende Beispiel 1.

Beispiel 1

5 µg DNA wird mit dem Restriktionsenzym Pst I komplett verdaut und nach Auftrennung in einem 0,8% Agarose-Gel auf Nitrocellulose-Filter übertragen (Southern-Transfer). Der Filter mit der gebundenen, denaturierten DNA wird 6 Stunden in 5 ml Vorhybridisierungsmedium (0,6 M NaCl, 0,06 M Na-EDTA, 0,1% Natriumdodecylsulfat-Lösung, 100 µg/ml ultraschallbehandelter Kalbsthymus-DNA und 4-fach konzentrierter Denhardt-Lösung) vorhybridisiert. Anschliessend wird erneut mit 5 ml des Vorhybridisierungsmediums behandelt, dem jedoch 500 000 cpm/ml radioaktiv markierter DNA zugesetzt sind. Diese radioaktiv markierte Probe wird wie folgt erhalten:

Die DNA-Sequenz A wird chemisch nach dem Phosphit-Verfahren synthetisiert. Sie enthält 20 Nucleotide (Mol-gewicht etwa 13 000) und ist komplementär zur putativen DNA-Sequenz für Tendamistat, abgeleitet von der Aminosäuresequenz von Tendamistat ab Aminosäure 37 des Tendamistats unter Gebrauch der von E. coli bevorzugten Tripletts. Diese DNA-Sequenz A wird an 5'-Ende mit Hilfe von $\gamma$-$^{32}$P-ATP und Nucleotid-Kinase radioaktiv markiert.

Zur Hybridisierung dieser radioaktiven Probe zur komplementären DNA-Sequenz in der Gesamt-DNA lässt man das Gemisch 24 Stunden bei 37°C stehen. Danach wird die nichtgebundene radioaktive DNA entfernt und der Filter bei 37°C 5 × jeweils 30 Minuten mit je 200 ml Hybridisierungsmedium gewaschen und anschliessend autoradiographiert. Nach 24 Stunden Exposition zeigen die Hybridisierungssignale, dass das Gen auf einem 2,3 kb Pst I-Fragment liegt. Dieses Fragment wird durch Elektroelution eines dieser Fragmentgrösse entsprechenden Anschnittes aus einem präparativen Agarose-Gel gewonnen,

auf dem PstI-verdaute Gesamt-DNA aufgetrennt wurde. Die eluierte DNA wird in die Pst I-Schnittstelle des Plasmids pUC 8 kloniert.

Diese Hybridplasmide werden in E. coli JM 103 transformiert und amplifiziert. Der Nachweis derjenigen Klone, die die gesuchte Insertion mit dem Tendamistat-Gen tragen, wird durch Kolonie-Hybridisierung unter Verwendung der radioaktiven DNA-Probe A geführt. Die so erhaltenen Hybridplasmide pKAI 1 a bzw. 1 b sind auf der Fig. 1 a und b dargestellt.

Durch weitere Southern-Hybridisierungen gegen das isolierte 2,3 kb Pst I-Fragment und dessen Subfragmente lässt sich die Lokalisation des Gens exakt ermitteln (Fig. 2a bis 2c).

Beispiel 2

In die singuläre Schnittstelle des Plasmids pIJ 102 für Pst I wird das 2,3 kb Pst I-Fragment aus S. tendae kloniert. Die so erhaltenen Hybridplasmide pAX 1a und 1b, die sich durch die Orientierung der Insertion unterscheiden, befähigen nach Transformation in S. lividans-Stämme diese zur Produktion von Tendamistat. Fig. 3 zeigt das Plasmid pAX 1a.

Beispiel 3

Der handelsübliche Stamm S. lividans TK 24 (John Innes Institute, Norwich, England) wird in bekannter Weise in Protoplasten überführt und 1 µg Hybridplasmid pAX 1a mit $10^8$ Protoplasten unter Zusatz von 20% Polyethylenglykol 6000 versetzt. Die transformierten Protoplasten werden auf Regenerationsmedium R2YE (Thompson et al., Nature 286 (1980) 525) bei 30°C 5 Tage bebrütet.

Der Nachweis, dass ein extrazellulärer Amylase-Inaktivator gebildet wurde, kann durch einen Plattentest erfolgen:

Die regenerierten Kolonien werden mit 5 ml einer 0,4 bis 1,0 mg/ml Pankreatin enthaltenden wässrigen Lösung übergossen und 1 Stunde bei 37°C inkubiert. Die Lösung wird dann entfernt und durch 5 ml eines 2%igen Stärke-Agars ersetzt. Nach 2 Stunden Inkubation bei 37°C werden die Platten mit 5 ml einer Jod-Kaliumjodid-Lösung zur Entwicklung übergossen. Kolonien mit einem blauen Hof zeigen an, dass die Klone Tendamistat synthetisieren und exkretieren.

Zur Kontrolle kann aus Tendamistat produzierenden, gut sporulierenden Stämmen die Plasmid-DNA isoliert und kartiert werden. Alle Tendamistat produzierenden Stämme weisen die eingesetzte Plasmid-DNA auf.

Beispiel 4

Man verfährt gemäss Beispiel 2, setzt jedoch das Plasmid pIJ 350 ein, das als Selektionsmarker in Streptomyceten ein Thiostrepton-Resistenzgen trägt. Man erhält so die Hybridplasmide pAX 350a und b (die sich durch die Orientierung der Insertion unterscheiden). Fig. 4 zeigt das Plasmid pAX 350a.

Nach Transformation gemäss Beispiel 3 werden auf Minimalmedium (Hopwood, Bacteriological Reviews 31 (1967) 373–403) in Gegenwart von 50 µg/ml Thiostrepton resistente Klone selektiert und entweder direkt auf Minimalmedium oder nach Umstempeln auf nicht selektiven R2YE-Agar auf Tendamistat-Produktion getestet.

Beispiel 5

Hybridplasmide, die das 2,3 kb Pst I-Fragment enthalten und ausser dem Streptomyceten-Replicon auch ein E. coli-Replicon enthalten, weisen als «Shuttle»-Vectoren eine Reihe von Vorteilen auf: Aufgrund des E. coli-Replicons und in E. coli wirksamen Resistenzmarkern können sie in diesen Organismen gut amplifiziert werden. Nach Isolierung und Transformation in Streptomyceten, insbesondere S. lividans, zeigen sie eine hohe Stabilität. In Folge ihrer im Streptomyceten wirksamen Selektionsmarker und des Streptomyceten-Replicons können sie auch in diesen Organismen gut amplifiziert werden und exprimieren und sekretieren Tendamistat.

Das Plasmid pAC 184 wird komplett mit dem Restriktionsenzym Sal I verdaut und das Enzym durch Phenol-Chloroform-Extraktion entfernt. Die überhängenden 5′-Enden werden in Gegenwart von ATP, CTP, GTP und TTP mit Hilfe des Enzyms DNA-Polymerase (Klenow-Fragment) aufgefüllt. An die stumpfen Enden (blunt ends) wird in einer Ligase-Reaktion bei 16°C ein Pst I-Linker der Struktur

5′TCG AGC TGC AGC TCG A 3′
3′AGC TCG ACG TCG AGC T 5′

ligiert (2 µg Linker auf 0,4 µg DNA). Die DNA wird mit Phenol-Chloroform extrahiert und nach Fällen mit dem Enzym Pst I zur Gewinnung ligierbarer Pst I-Enden verdaut. Die DNA wird anschliessend dephosphoryliert, erneut mit Phenol-Chloroform extrahiert und mit partiell Pst I-verdautem Plasmid pAX 1a ligiert. Das Ligationsgemisch wird in E. coli (HB 101 bzw. MC 1061) transformiert. Gegen Chloramphenicol resistente Klone werden von der Platte abgeschwemmt und die DNA isoliert. S. lividans TK 24 wird mit 1 bis 2 µg DNA transformiert und auf Tendamistat-Produktion hin geprüft.

Im Tendamistat-Test positiv reagierende Klone werden isoliert, die Plasmid-DNA durch alkalische Schnell-Lyse isoliert und in E. coli HB 101 bzw. MC 1061 rücktransformiert. Nach Amplifizierung reisolierte Plasmide unterscheiden sich nicht von den aus S. lividans-Stämmen isolierten Plasmiden. Je nach Orientierung der Insertion, die das Tendamistat-Gen trägt, werden die rekombinanten Plasmide als pSA 2a oder b bezeichnet (Fig. 5a und b).

Beispiel 6

Verfährt man gemäss Beispiel 5, geht jedoch vom Plasmid pAX 350a aus, selektioniert in E. coli auf Chloramphenicol-Resistenz und in S. lividans auf Thiostrepton-Resistenz und Tendamistat-Produktion, so erhält man die Plasmide pSA 351a bzw. b (Fig. 6a/b).

Beispiel 7

Verfährt man gemäss Beispiel 5, geht jedoch an Stelle des Plasmids pAC 184 von PAC 177 aus, so erhält man die Plasmide pSA 3a bzw. b (Fig. 7a/b).

Hierzu wird das Plasmid pAX 1 partiell mit Pst I geschnitten und das Enzym durch 15minütiges Erhitzen auf 68°C hitzeinaktiviert. Die DNA wird in das mit Pst I geschnittene dephosphorylierte und deproteinierte Plasmid pAC 177 ligiert. Nach Transformation von E. coli HB 101 bzw. MC 1061 werden gegen Kanamycin resistente Klone von der Platte abgeschwemmt, die Plasmid-DNA isoliert und S. lividans TK 24 mit 1 bis 2 µg dieser DNA transformiert. Tendamistat produzierende Klone werden selektioniert und die Plasmide charakterisiert.

Beispiel 8

Verfährt man gemäss Beispiel 7, setzt jedoch an Stelle des Plasmids pAX 1 das Plasmid pAX 350 a ein und selektioniert in S. lividans auf Thiostrepton-Resistenz, so erhält man die Plasmide pSA 352a bzw. b (Fig. 8a/b).

Beispiel 9

Wie sich aus Fig. 2 ergibt, ist das für Tendamistat und die Signalsequenz codierende Gen ~ 0,3 kb gross. Das in den vorstehend beschriebenen Beispielen eingesetzte 2,3 kb-Fragment kann also in verkürzter Form zur Konstruktion von Hybridplasmiden eingesetzt werden, die die Produktion von Tendamistat bewirken:

Das Plasmid pKAI 1 a wird mit Sal I verdaut und religiert. Man erhält so das um ca. 750 Basenpaare verkürzte Plasmid pKAI 2. Dieses wird kloniert, isoliert und mit Pst I geschnitten. Die DNA wird mit alkalischer Phosphatase aus Kälberdarm dephosphoryliert und mit Phenol-Chloroform deproteinisiert.

Das Plasmid pIJ 102 wird komplett mit Pst I geschnitten und die Fragmente nach Hitze-Inaktivierung des Enzyms in die Pst I-Schnittstelle von pKAI 2 ligiert. Das Ligationsgemisch wird in E. coli HB 101 oder MC 1061 transformiert. Die Plasmid-DNA aus gegen Ampicillin resistenten Klonen wird durch alkalische Schnell-Lyse-Verfahren isoliert und S. lividans TK 24 mit 1 bis 2 µg dieser DNA transformiert. Man selektioniert auf Tendamistat-Produktion und isoliert die Plasmid-DNA dieser Klone durch alkalische Schnell-Lyse. Durch Rücktransformation in E. coli HB 101 bzw. MC 1061 und nach Isolierung der Plasmid-DNA aus den transformierten E. coli-Stämmen charakterisiert man das Plasmid pSA 1 durch Restriktionsanalyse (Fig. 9). Das Plasmid zeigt keinen Unterschied zu den aus S. lividans-Stämmen isolierten Plasmiden, jedoch ist die DNA-Aufarbeitung aus E. coli ergiebiger und in kürzerer Zeit möglich.

Beispiel 10

Verfährt man gemäss Beispiel 9, setzt jedoch an Stelle des Plasmids pIJ 102 das Plasmid pIJ 350 ein, wobei in S. lividans dann zusätzlich nach der Thiostrepton-Resistenz selektioniert werden kann, so erhält man die Plasmide pSA 350a bzw. b. Die Fig. 10 zeigt das Plasmid pSA 350a. Dieses Plasmid führt in der Schüttelkultur zu höheren Ausbeuten an Tendamistat als das Plasmid pSA 350b, das die Insertion mit dem Tendamistat-Gen in umgekehrter Orientierung enthält. Hingegen hat die Verkleinerung der Insertion auf 1,5 kb keinen signifikanten Einfluss auf die Produktbildung.

Beispiel 11

Zur Ermittlung der Struktur und Nucleotidsequenz des Tendamistat-Gens wurde das 0,94 kb Pst I-Bam HI-Subfragment (Fig. 2a und b) sowie das 295 bp Sau 3a-Bam HI-Subfragment (Fig. 2c) in die Einzelstrangphagen M 13 mp 8 und M 13 mp 9 kloniert. Als Primer für die Di-Deoxy-Sequenzierreaktion diente die 20 Nucleotide umfassende DNA-Sequenz A sowie ein kommerziell erhältlicher 15 bp-Primer (Bethesda Research Laboratories GmbH, Neu-Isenburg). Es wurde die DNA-Sequenz C ermittelt.

Beispiel 12

Vor dem Strukturgen von Tendamistat befindet sich auf der DNA ein offener Leserahmen bis zum Startcodon ATG (Met) für ein Protein, das dem aminoterminalen Ende von Tendamistat nahtlos vorgelagert ist. Diesem Signalpeptid entspricht die DNA-Sequenz B.

Anhang:

DNA-Sequenz C (codierender Strang)

5′-GAC ACG ACC GTC TCC GAG CCC GCA CCC TCC TGC GTG $NH_2$-Asp Thr Thr Val Ser Glu Pro Ala Pro Ser Cys Val

ACG CTC TAC CAG AGC TGG CGG TAC TCA CAG GCC GAC Thr Leu Tyr Gln Ser Trp Arg Tyr Ser Gln Ala Asp

AAC GGC TGT GCC GAG ACG GTG ACC GTG AAG GTC GTC Asn Gly Cys Ala Glu Thr Val Thr Val Lys Val Val

TAC GAG GAC GAC ACC GAA GGC CTG TGC TAC GCC GTC Tyr Glu Asp Asp Thr Glu Gly Leu Cys Tyr Ala Val

GCA CCG GGC CAG ATC ACC ACC GTC GGC GAC GGC TAC Ala Pro Gly Gln Ile Thr Thr Val Gly Asp Gly Tyr

ATC GGC TCG CAC GGC CAC GCG CGC TAC CTG GCT CGC Ile Gly Ser His Gly His Ala Arg Tyr Leu Ala Arg

TGC CTT TAG-3′ Cys Leu Stp

**Patentansprüche**

für die Vertragsstaaten BE. CH. DE. FR. GB. IT. LI. LU. NL. SE

1. DNA-Sequenz B, erhältlich aus Tendamistat-produzierenden Streptomyces tendae-Stämmen, die vorzugsweise mit sublethalen Dosen Acriflavin behandelt wurden, durch Isolieren der Gesamt-DNA, Verdauen mit Pst I, Southern-Hybridisierung mit der DNA-Sequenz A

5′-($^{32}$P-)CCT TCA GTG TCG TCT TCG TA-3′ (A)

Isolieren des 2,3 kb-Pst I-Fragments, Schneiden mit BamHI, Southern-Hybridisieren mit der Sequenz A, Isolieren des 0,94 kb-Pst I-BamHI-Subfragments, Schneiden mit Sau 3a, Southern-Hybridisieren mit der Sequenz A, Isolieren des 0,525 kb-BamHI-Sau 3a-Subfragments und Sequenzierung der DNA, gekennzeichnet durch die Merkmale:

a) sie liegt unmittelbar vor dem Tendamistat-Strukturgen,

b) sie codiert am Amino-Terminus für Met-Arg-Val-Arg-Ala-Leu-Arg,

c) sie codiert am Carboxy-Terminus für Ala-Ser-Ala und

d) sie codiert in der Mitte für einen hydrophoben Bereich X, der 10 bis 25, vorzugsweise 17 bis 20, Aminosäuren umfasst.

2. Peptid der allgemeinen Formel Met-Arg-Val-Arg-Ala-Leu-Arg-X-Ala-Ser-Ala-R in der R Hydroxy oder den Rest eines genetisch codierbaren Peptids bedeutet und X für den im Anspruch 1 definierten hydrophoben Bereich steht.

3. Genstruktur, enthaltend die im Anspruch 1 definierte DNA-Sequenz B, vorzugsweise unmittelbar im Leserahmen mit einem Strukturgen, insbesondere dem Strukturgen für Tendamistat, vor allem der DNA-Sequenz C.

4. Plasmid, gekennzeichnet durch eine DNA-Sequenz nach Anspruch 1 oder 3, vorzugsweise mit einem in Streptomyceten wirksamen Replicon und/oder einem in E. coli wirksamen Replicon.

5. Wirtsorganismus der Gattung Streptomyces, insbesondere der Art S. tendae oder S. lividans, enthaltend ein Plasmid nach Anspruch 4.

6. Verfahren zur Herstelung eines Polypeptids, dadurch gekennzeichnet, dass man einen Wirtsorganismus nach Anspruch 5 einsetzt, der eine Signalpeptidase enthält, welche das der DNA-Sequenz B entsprechende Präpeptid abtrennt.

7. Verfahren nach Anspruch 6, dadurch gekennzeichnet, dass das Polypeptid nach Abtrennung des Präpeptids aus der Zelle ausgeschleust wird.

8. Verfahren nach Anspruch 6 oder 7, dadurch gekennzeichnet, dass man einen Wirtsorganismus mit einem Plasmid einsetzt, das eine Genstruktur nach Anspruch 3 enthält.

**Patentansprüche**

für den Vertragsstaat AT:

1. Verfahren zur Herstellung von Polypeptiden aus genetisch codierbaren Aminosäuren, dadurch gekennzeichnet, dass man in einer Wirtszelle der Gattung Streptomyces eine Genstruktur exprimiert, die im Leserahmen mit dem Strukturgen für das gewünschte Polypeptid die DNA-Sequenz B eines Präpeptids enthält, welche aus Tendamistat-produzierenden Streptomyces tendae Stämmen, die vorzugsweise mit sublethalen Dosen Acriflavin behandelt wurden, durch Isolieren der Gesamt-DNA, Verdauen mit Pst I, Southern-Hybridisierung mit der DNA-Sequenz A

5′-($^{32}$P-) CCT TCA GTG TCG TCT TCG TA-3′ (A)

Isolieren des 2,3 kb-Pst I-Fragments, Schneiden mit BamHi, Southern-Hybridisieren mit der Sequenz A, Isolieren des 0,94 kb-Pst I-BamHI-Subfragments, Schneiden mit Sau 3a, Southern-Hybridisieren mit der Sequenz A, Isolieren des 0,525 kb-BamHI-Sau 3a-Subfragments und Sequenzierung der DNA erhältlich ist und

a) unmittelbar vor dem Strukturgen liegt,

b) am Amino-Terminus für Met-Arg-Val-Arg-Ala-Leu-Arg codiert,

c) am Carboxy-Terminus für Ala-Ser-Ala codiert und

d) in der Mitte für einen hydrophoben Bereich X codiert, der 10 bis 25 Aminosäuren umfasst.

2. Verfahren nach Anspruch 1, dadurch gekennzeichnet, dass die DNA-Sequenz B des Präpeptids in der Mitte für einen hydrophoben Bereich X codiert, der 17 bis 20 Aminosäuren umfasst.

3. Verfahren nach Anspruch 1 oder 2, dadurch gekennzeichnet, dass die DNA-Sequenz B für das Präpeptid aus S. tendae isoliert ist.

4. Verfahren nach Anspruch 1 bis 3, dadurch gekennzeichnet, dass die Wirtszelle von der Art S. tendae oder S. lividans ist.

5. Verfahren nach Anspruch 1 bis 4, dadurch gekennzeichnet, dass die Wirtszelle eine Signalpeptidase enthält, welche das Präpeptid abspaltet und das gewünschte Polypeptid in das Kulturmedium ausscheidet.

6. Verfahren nach einem oder mehreren der vorhergehenden Ansprüche, dadurch gekennzeichnet, dass die Genstruktur in die Wirtszelle mit einem Plasmid eingebracht wird, das ein in Streptomyceten wirksames Replicon enthält.

7. Verfahren nach Anspruch 6, dadurch gekennzeichnet, dass das Plasmid ausser dem in Streptomyceten wirksamen Replicon ein in E. coli wirksames Replicon enthält.

8. Verfahren nach einem oder mehreren der vorhergehenden Ansprüche, dadurch gekennzeichnet, dass das Strukturgen für Tendamistat codiert.

9. Verfahren nach Anspruch 8, dadurch gekennzeichnet, dass das Sturkturgen die DNA-Sequenz C (Anhang) aufweist.

**Claims**

for the contracting states: BE. CH. LI. DE. FR. GB. IT. LU. NL. SE

1. DNA sequence B, obtainable from Streptomyces tendae strains, which produce tendamistat and have preferably been treated with sublethal doses of acriflavine, by isolation of the complete DNA, digestion with Pst I, Southern hybridization with the DNA sequence A,

5′-($^{32}$P-) CCT TCA GTG TCG TCT TCG TA-3′ (A)

isolation of the 2.3 kb Pst I fragment, cutting with BamHI, Southern hybridization with the sequence A, isolation of the 0.94 kb Pst I-BamHI subfragment, cutting with Sau 3a, Southern hybridization with the sequence A, isolation of the 0.525 kb BamHI-Sau 3a subfragment and sequencing of the DNA, and which has the following features:

a) it is located immediately upstream of the tendamistat structural gene,

b) it codes at the amino terminal end for Met-Arg-Val-Arg-Ala-Leu-Arg,

c) it codes at the carboxyl terminal end for Ala-Ser-Ala and

d) it codes in the middle for a hydrophobic region X which comprises 10 to 25, preferably 17 to 20, amino acids.

2. A peptide of the general formula Met-Arg-Val-Arg-Ala-Leu-Arg-X-Ala-Ser-Ala-R in which R denotes hydroxyl or the residue of a genetically codable peptide, and X represents the hydrophobic region defined in claim 1.

3. A gene structure containing the DNA sequence B defined in claim 1, preferably directly in the reading frame with a structural gene, in particular the structural gene for tendamistat, especially DNA sequence C.

4. A plasmid having a DNA sequence as claimed in claim 1 or 3, preferably having a replicon effective in Streptomycetes and/or having a replicon effective in E. coli.

5. A host organism of the genus Streptomyces, especially of the species S. tendae or S. lividans, containing a plasmid as claimed in claim 4.

6. A process for the preparation of a polypeptide, characterized by using a host organism as claimed in claim 5, which contains a signal peptidase which detaches the prepeptide corresponding to the DNA sequence B.

7. The process as claimed in claim 6, characterized in that the polypeptide is, after detachment of the prepeptide, released from the cell.

8. The process as claimed in claim 6 or 7, chracterized in that a host organism having a plasmid which contains a gene structure as claimed in claim 3 is used.

## Claims

for contracting state AT

1. A process for the preparation of polypeptides composed of genetically codable amino acids, characterized by the expression, in a host cell of the genus Streptomyces, of a gene structure which contains in the reading frame with the structural gene for the desired polypeptide the DNA sequence B of a prepeptide, which sequence is obtainable from Streptomyces tendae strains, which produce tendamistat and have preferably been treated with sublethal doses of acriflavine, by isolation of the complete DNA, digestion with Pst I, Southern hybridization with the DNA sequence A,

5'-($^{32}$P-)CCT TCA GTG TCG TCT TCG TA-3' (A)

isolation of the 2.3 kb Pst I fragment, cutting with BamHI, Southern hybridization with the sequence A, isolation of the 0.94 kb Pst I-BamHI subfragment, cutting with Sau 3a, Southern hybridization with the sequence A, isolation of the 0.525 kb BamHI-Sau 3a subfragment and sequencing of the DNA, and

a) is located immediately upstream of the structural gene,

b) codes at the amino terminal end for Met-Arg-Val-Arg-Ala-Leu-Arg,

c) codes at the carboxyl terminal end for Ala-Ser-Ala and

d) codes in the middle for a hydrophobic region X which comprises 10 to 25 amino acids.

2. The process as claimed in claim 1, characterized in that the DNA sequence B of the prepeptide codes in the middle for a lydrophobic region X which comprises 17 to 20 amino acids.

3. The process as claimed in'claim 1 or 2, characterized in that the DNA sequence B for the prepeptide is isolated from S. tendae.

4. The process as claimed in claims 1 to 3, characterized in that the host cell is of the species S. tendae or S. lividans.

5. The process as claimed in claims 1 to 4, characterized in that the host cell contains a signal peptidase which cleaves off the prepeptide, and secretes the desired polypeptide into the culture medium.

6. The process as claimed in one or more of the preceding claims, characterized in that the gene structure is introduced into the host cell with a plasmid which contains a replicon effective in Streptomycetes.

7. The process as claimed in claim 6, characterized in that the plasmid has a replicon effective in E. coli apart from the replicon effective in Streptomycetes.

8. The process as claimed in one or more of the preceding claims, characterized in that the structural gene codes for tendamistat.

9. The process as claimed in claim 8, characterized in that the structural gene has the DNA sequence C (Annex).

## Revendications

pour les Etats contractant: BE. CH. DE. FR. GB. IT. LI. LU. NL. SE

1. Séquence d'ADN B, pouvant être obtenue, à partir de souches de Streptomyces tendae productrices de tendamistat qui ont de préférence été traitées avec des doses sublétales d'acriflavine, par isolement de l'ADN total, digestion par Pst I, hybridation de Southern avec la séquence d'ADN A

5'-($^{32}$P-)CCT TCA GTG TCG TCT TCG TA-3' (A),

isolement du fragment Pst I de 2,3 kb, coupure par Bam HI, hybridation de Southern avec la séquence A, isolement du sous-fragment Pst I-Bam HI de 0,94 kb, coupure par Sau 3a, hybridation de Southern avec la séquence A, isolement du sous-fragment Bam HI-Sau 3a de 0,525 kb, et séquençage de l'ADN, caractérisée en ce que:

a) elle se trouve immédiatement avant le gène structural du tendamistat,

b) au niveau de la terminaison amino, elle code pour Met-Arg-Val-Arg-Ala-Leu-Arg,

c) au niveau de la terminaison carboxy, elle code pour Ala-Ser-Ala et

d) dans sa partie médiane, elle code pour une zone hydrophobe X qui comprend de 10 à 25, de préférence 17 à 20 acides aminés.

2. Peptide de formule générale Met-Arg-Val-Arg-Ala-Leu-Arg-X-Ala-Ser-Ala-R dans lequel R représente un groupe hydroxy ou le reste d'un peptide codable génétiquement, et X représente la zone hydrophobe définie dans la revendication 1.

3. Structure génique contenant la séquence d'ADN B définie dans la revendication 1, de préférence immédiatement dans le cadre de lecture comportant un gène structural, notamment le gène structural codant pour le tendamistat, en particulier la séquence d'ADN C.

4. Plasmide caractérisé par une séquence d'ADN selon la revendication 1 ou 3, de préférence comportant un réplicon actif dans des Streptomyces et/ou un réplicon actif dans E. coli.

5. Organisme hôte du genre Streptomyces, en particulier du type S. tendae ou S. lividans, contenant un plasmide selon la revendication 4.

6. Procédé pour l'obtention d'un polypeptide, caractérisé en ce que l'on utilise un organisme hôte selon la revendication 5, lequel contient une signal-peptidase qui scinde le prépeptide correspondant à la séquence d'ADN B.

7. Procédé selon la revendication 6, caractérisé en ce que le polypeptide est excrété hors de la cellule après scission du prépeptide.

8. Procédé selon la revendication 6 ou 7, caractérisé en ce que l'on utilise un organisme hôte comportant un plasmide qui contient une structure génique selon la revendication 3.

## Revendications

Pour l'Etat Contractant AT

1. Procédé pour l'obtention de polypeptides consistant d'acides aminés codables génétiquement, caractérisé en ce que l'on exprime dans une cellule hôte du genre Streptomyces une structure génique qui contient, dans le cadre de lecture comportant le gène structural codant pour le polypeptide recherché, la séquence d'ADN B d'un prépeptide, laquelle peut être obtenue à partir de souches de Streptomyces tendae productrices de tendamistat, qui ont de préférence été traitées avec des doses sublétales d'acriflavine, par isolement de l'ADN total, digestion par Pst I, hybridation de Southern avec la séquence d'ADN A

5'-($^{32}$P-)CCT TCA GTG TCG TCT TCG TA-3' (A),

isolement du fragment Pst I de 2,3 kb, coupure par Bam HI, hybridation de Southern avec la séquence A, isolement du sous-fragment Pst I-Bam HI de 0,94 kb, coupure par Sau 3a, hybridation de Southern avec la séquence A, isolement du sous-fragment Bam HI-Sau 3a de 0,525 kb, et séquençage de l'ADN, et

a) se trouve immédiatement avant le gène structural du tendamistat,

b) au niveau de la terminaison amino, code pour Met-Arg-Val-Arg-Ala-Leu-Arg,

c) au niveau de la terminaison carboxy, code pour Ala-Ser-Ala et

d) dans sa partie médiane, code pour une zone hydrophobe X qui comprend de 10 à 25 acides aminés.

2. Procédé selon la revendication 1, caractérisé en ce que la séquence d'ADN B du prépeptide code en sa partie médiane pour une zone hydrophobe X qui comprend de 17 à 20 acides aminés.

3. Procédé selon la revendication 1 ou 2, caractérisé en ce que la séquence d'ADN B codant pour le prépeptide est isolée à partir de S. tendae.

4. Procédé selon la revendication 1 à 3, caractérisé en ce que la cellule hôte est du type S. tendae ou S. lividans.

5. Procédé selon la revendication 1 à 4, caractérisé en ce que la cellule hôte contient une signal-peptidase qui scinde le prépetide et qui libère dans le milieu de culture le polypeptide recherché.

6. Procédé selon une ou plusieurs des revendications précédentes, caractérisé en ce que l'on introduit la structure génique dans la cellule hôte avec un plasmide qui contient un réplicon actif dans des Streptomyces.

7. Procédé selon la revendication 6, caractérisé en ce que le plasmide contient, outre le réplicon actif dans des Streptomyces, un réplicon actif dans E. coli.

8. Procédé selon une ou plusieurs des revendications précédentes, caractérisé en ce que le gène structural code pour le tendamistat.

9. Procédé selon la revendication 8, caractérisé en ce que le gène structural présente la séquence d'ADN C (appendice).

FIG. 1a

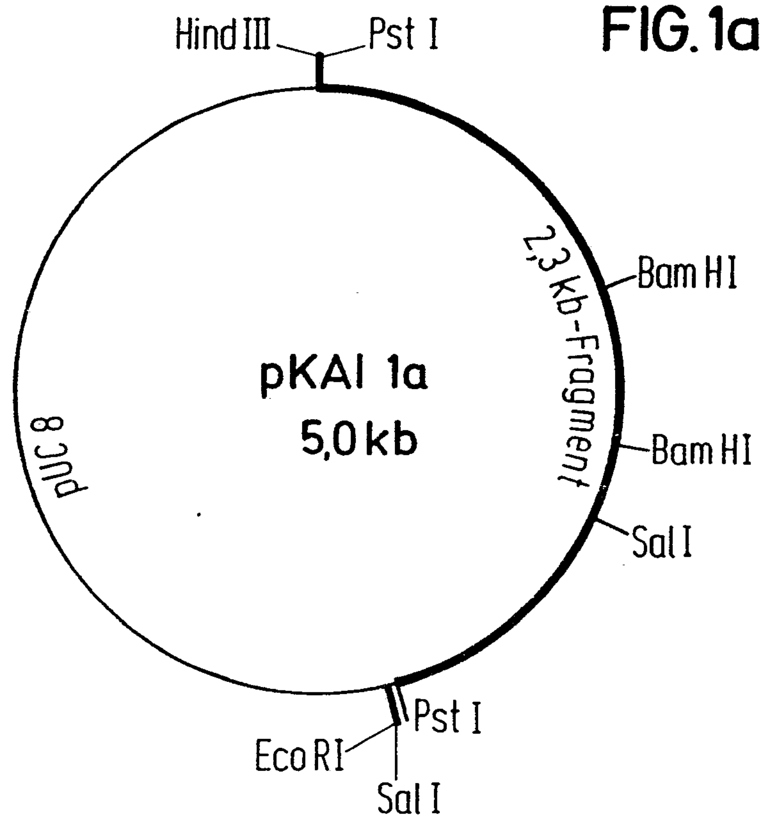
HindIII
Pst I
2,3 kb-Fragment
BamHI
BamHI
SalI
pKAI 1a
5,0kb
pUC 8
Pst I
EcoRI
Sal I

FIG. 1b

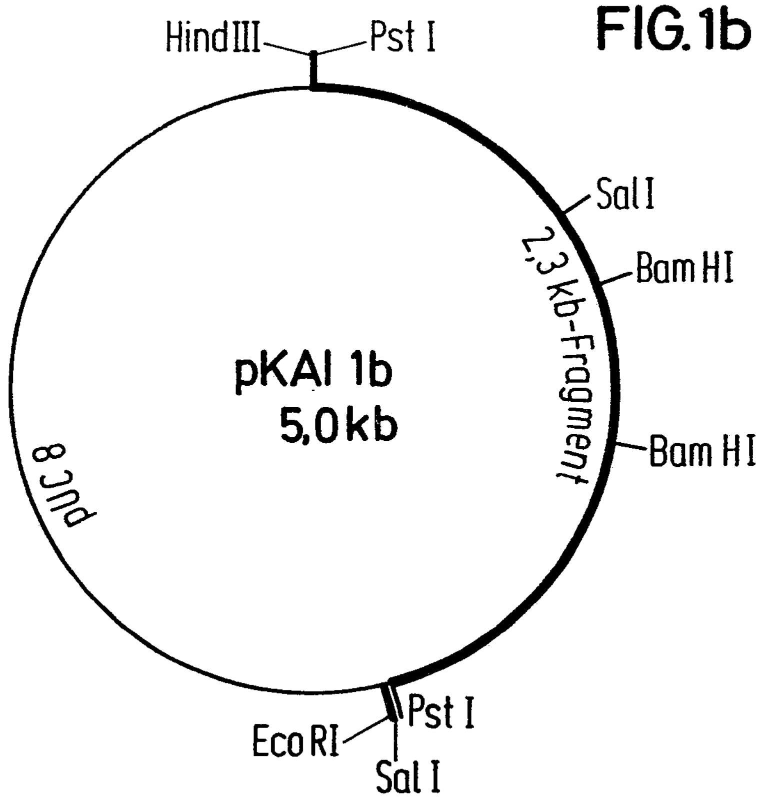
HindIII
Pst I
SalI
2,3 kb-Fragment
BamHI
BamHI
pKAI 1b
5,0kb
pUC 8
Pst I
EcoRI
Sal I

FIG. 2a

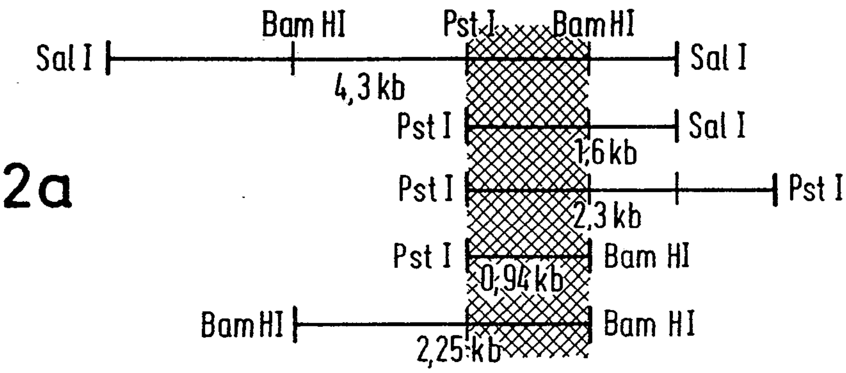


FIG. 2b

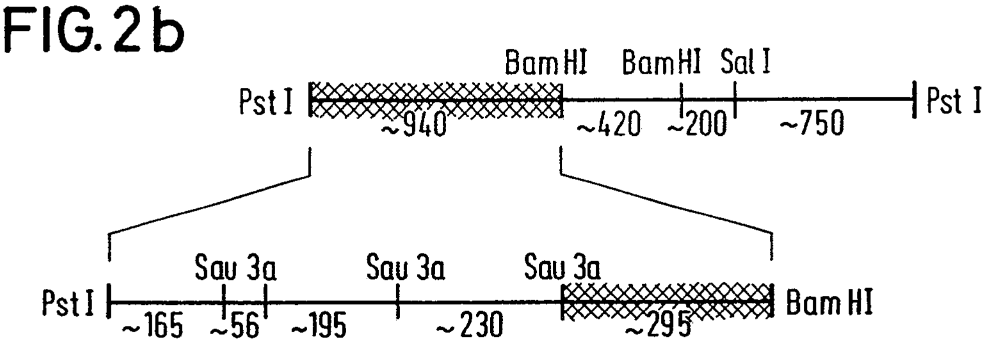


FIG. 2c

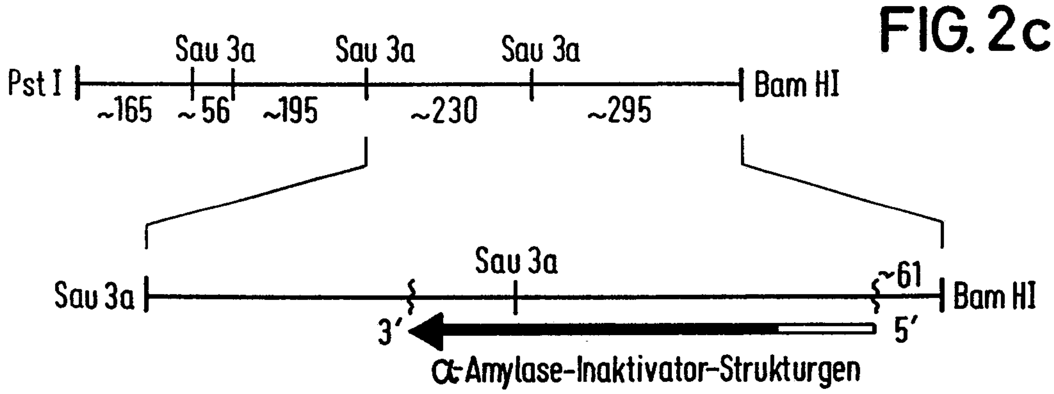

FIG. 3

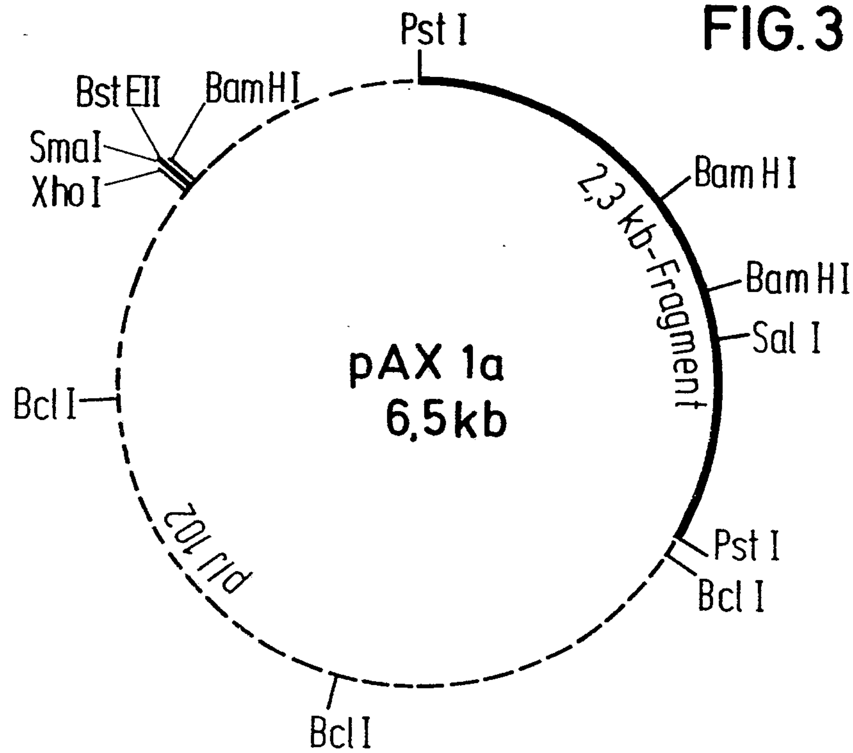
Pst I
Bst EII
Bam HI
Sma I
Xho I
Bam HI
2,3 kb-Fragment
Bam HI
Sal I
pAX 1a
6,5 kb
Bcl I
pIJ 102
Pst I
Bcl I
Bcl I

FIG. 4

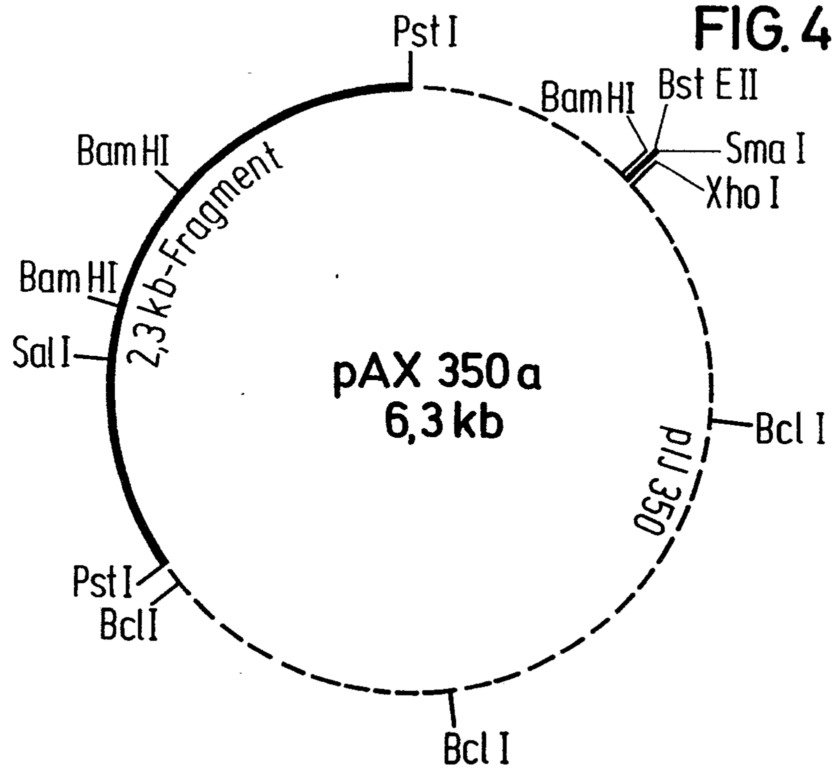
Pst I
Bam HI
Bst E II
Sma I
Xho I
Bam HI
2,3 kb-Fragment
Bam HI
Sal I
pAX 350 a
6,3 kb
Bcl I
pIJ 350
Pst I
Bcl I
Bcl I

FIG. 5a

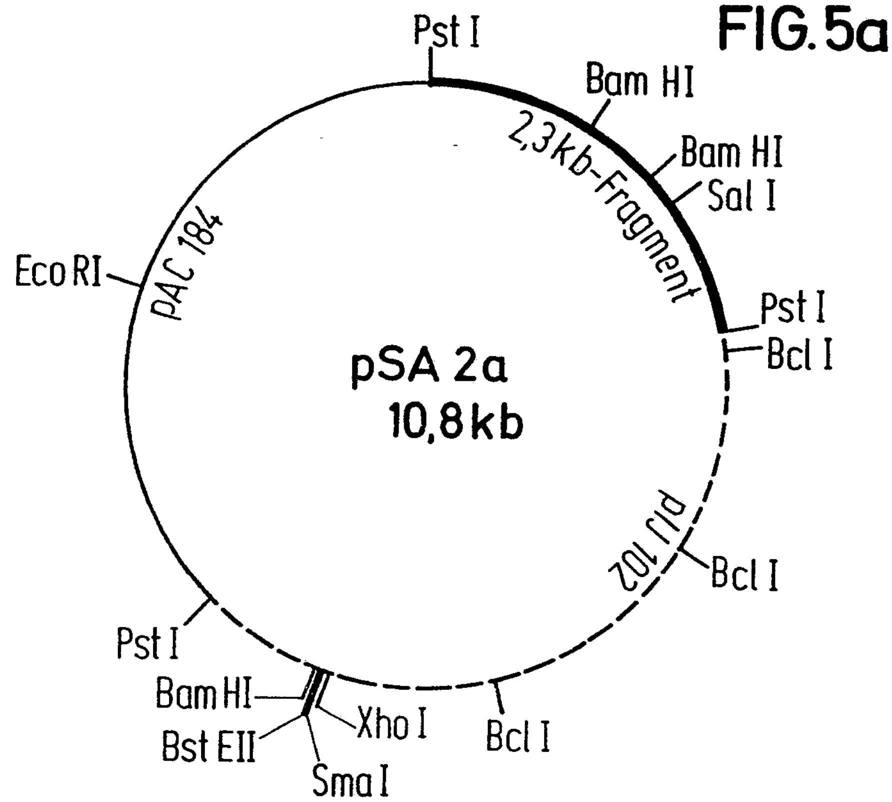
Pst I
Bam HI
Bam HI
Sal I
2,3 kb-Fragment
Pst I
Bcl I
Eco RI
pAC 184
pSA 2a
10,8 kb
pIJ 102
Bcl I
Pst I
Bam HI
Xho I
Bcl I
Bst EII
Sma I

FIG. 5b

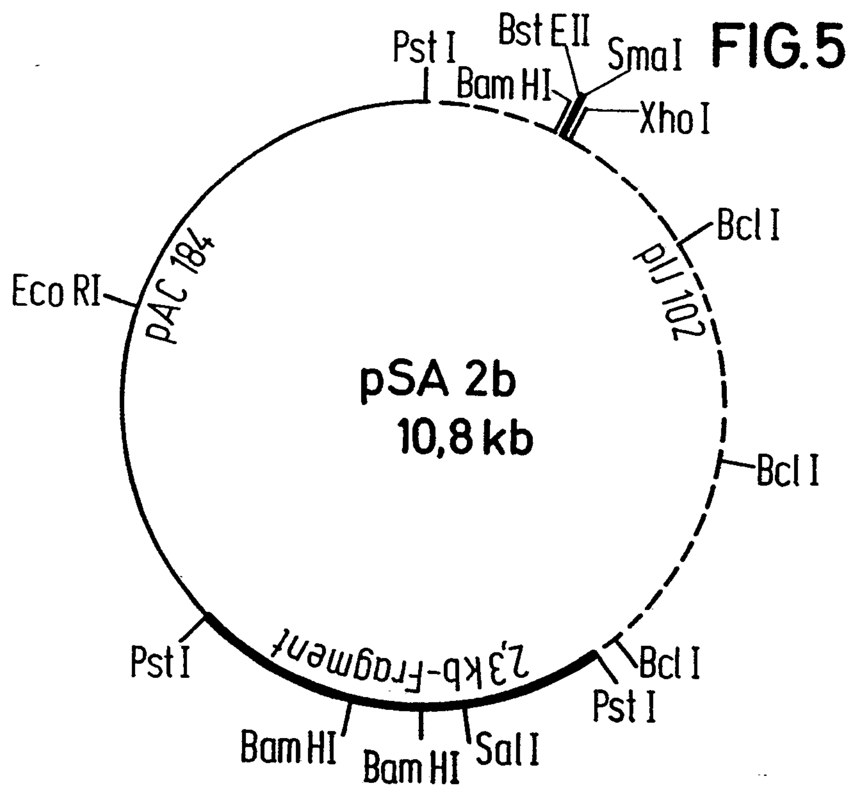
Pst I
Bst EII
Sma I
Bam HI
Xho I
Bcl I
Eco RI
pAC 184
pIJ 102
pSA 2b
10,8 kb
Bcl I
Pst I
2,3 kb-Fragment
Bcl I
Pst I
Bam HI
Bam HI
Sal I

FIG. 6a

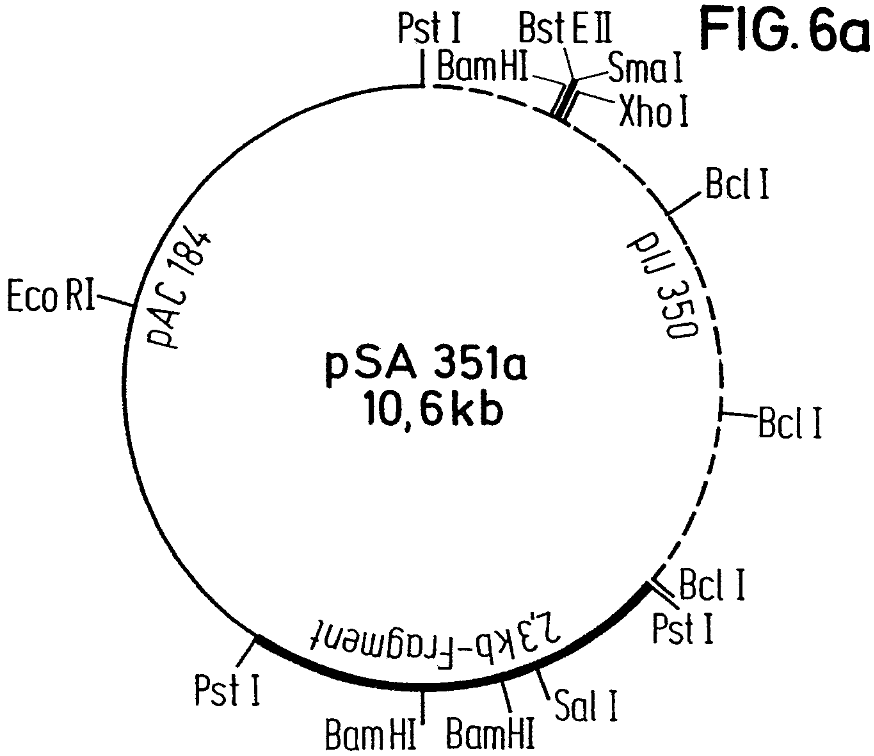
Pst I
Bst E II
Bam HI
Sma I
Xho I
Bcl I
pIJ 350
Bcl I
Bcl I
Pst I
Sal I
Bam HI
Bam HI
Pst I
2,3 kb-Fragment
Eco RI
pAC 184
pSA 351a
10,6 kb

FIG. 6b

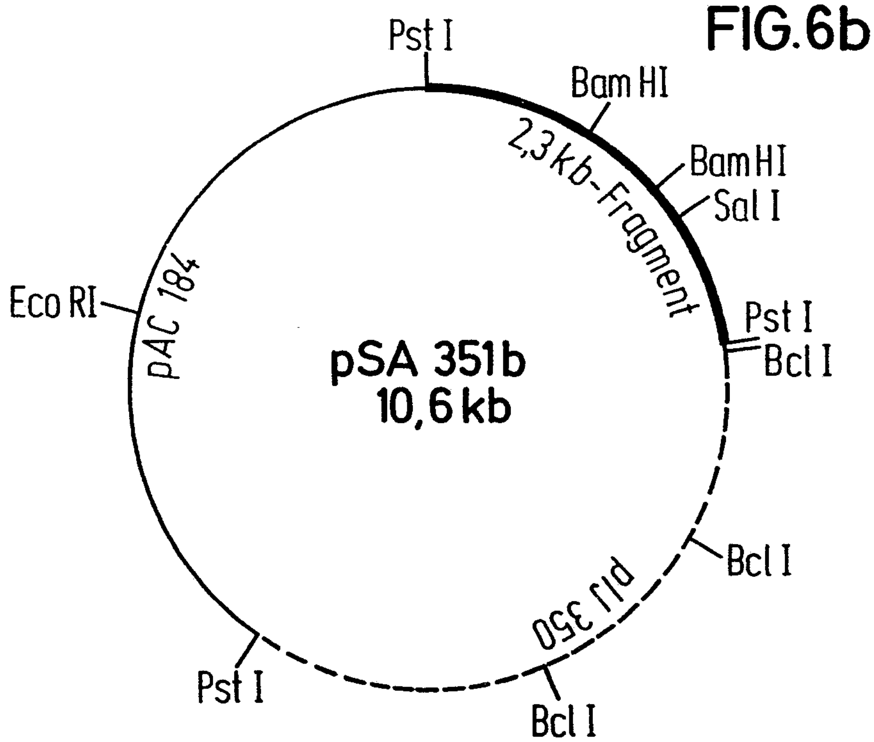
Pst I
Bam HI
Bam HI
Sal I
2,3 kb-Fragment
Pst I
Bcl I
Bcl I
pIJ 350
Bcl I
Pst I
Eco RI
pAC 184
pSA 351b
10,6 kb

FIG. 7a

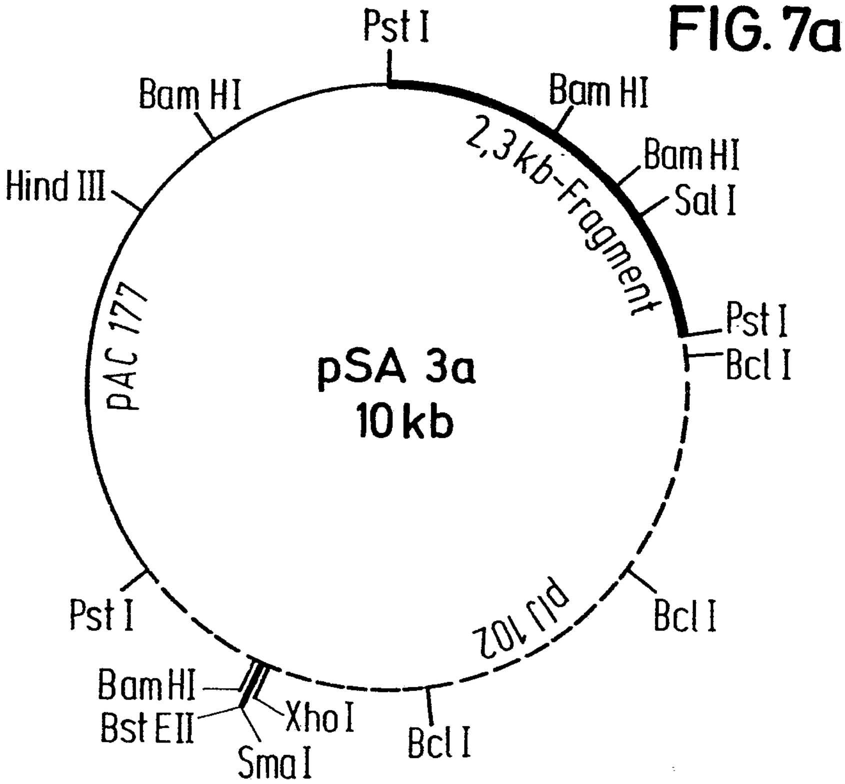
Pst I
Bam HI
2,3kb-Fragment
Bam HI
Sal I
Pst I
Bcl I
Bam HI
Hind III
pAC 177
pSA 3a
10 kb
Pst I
pIJ 102
Bcl I
Bam HI
Bst EII
Xho I
Sma I
Bcl I

FIG.7b

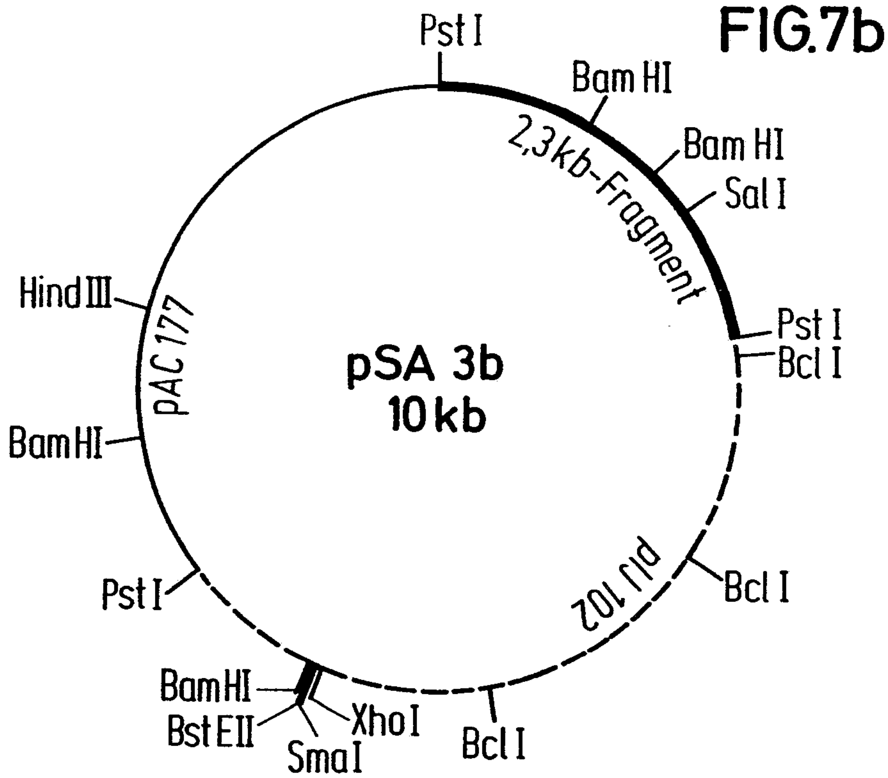
Pst I
Bam HI
2,3kb-Fragment
Bam HI
Sal I
Hind III
pAC 177
pSA 3b
10 kb
Pst I
Bcl I
Bam HI
Pst I
pIJ 102
Bcl I
Bam HI
Bst EII
Xho I
Sma I
Bcl I

FIG. 8a

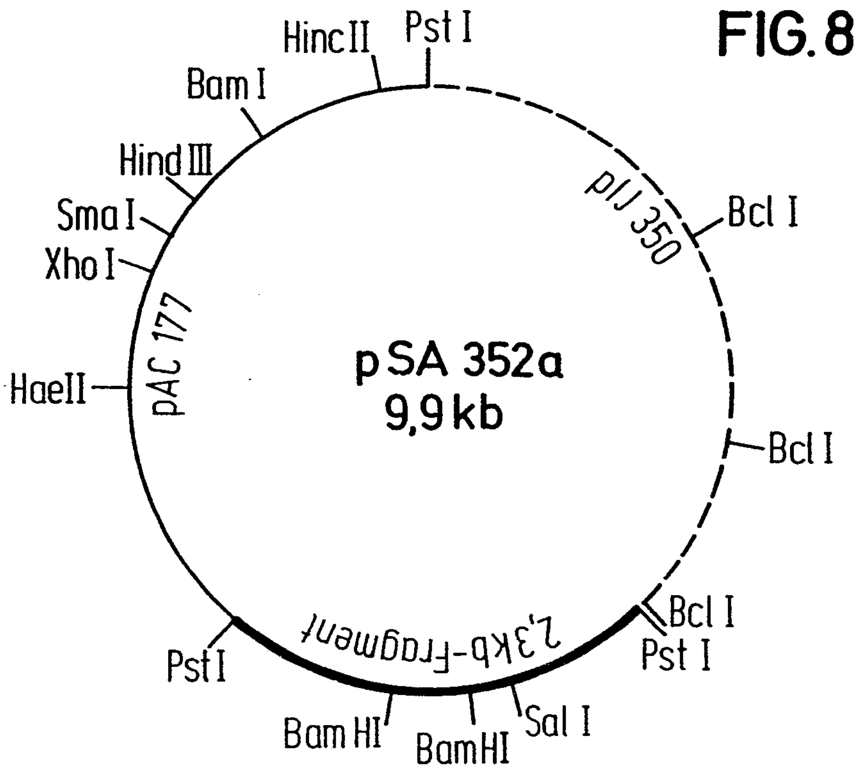
Hinc II
Pst I
Bam I
Hind III
Sma I
Xho I
Hae II
pAC 177
pIJ 350
Bcl I
Bcl I
pSA 352a
9,9 kb
Bcl I
Pst I
2,3 kb-Fragment
Pst I
Bam HI
Bam HI
Sal I

FIG. 8b

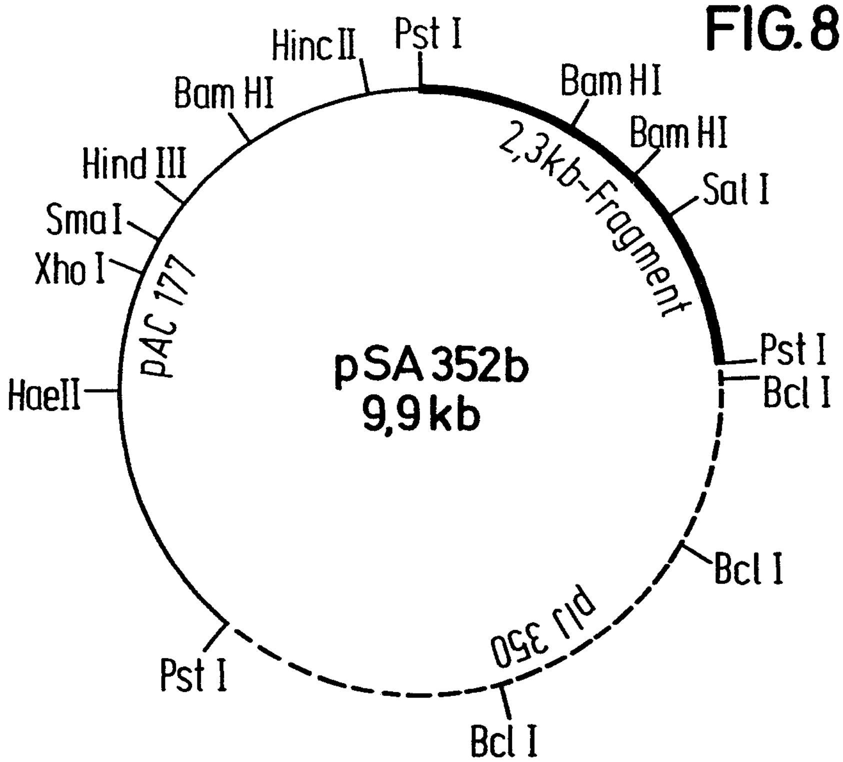
Hinc II
Pst I
Bam HI
Hind III
Sma I
Xho I
Hae II
pAC 177
Bam HI
Bam HI
2,3 kb-Fragment
Sal I
Pst I
Bcl I
pSA 352b
9,9 kb
Bcl I
pIJ 350
Pst I
Bcl I

FIG. 9

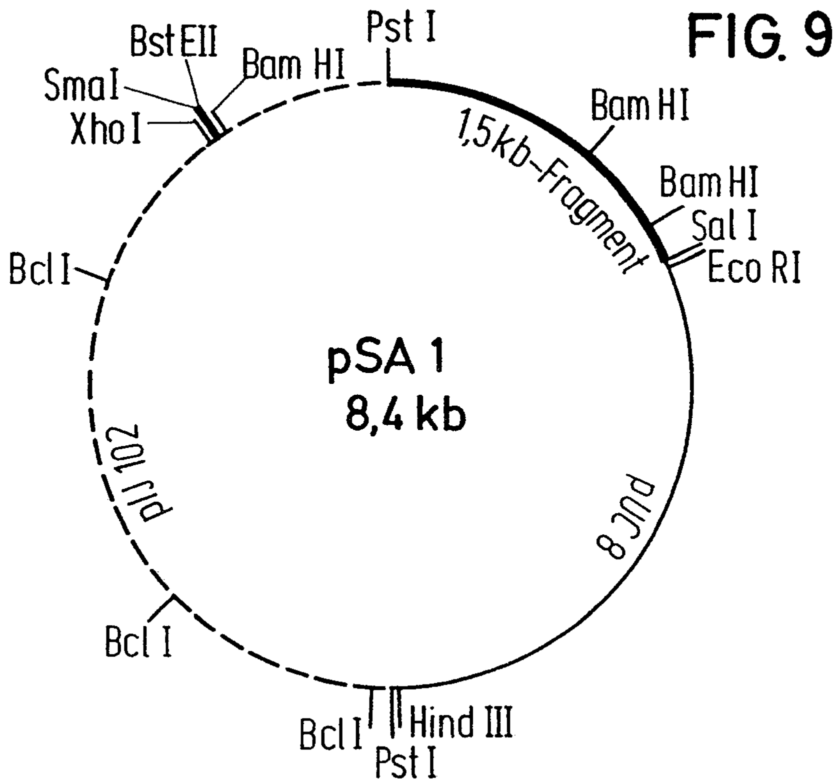

Pst I
Bst EII
Bam HI
Sma I
Xho I
Bam HI
1,5kb-Fragment
Bam HI
Sal I
Eco RI
Bcl I
pSA 1
8,4 kb
pIJ 102
pUC 8
Bcl I
Bcl I
Hind III
Pst I


FIG. 10

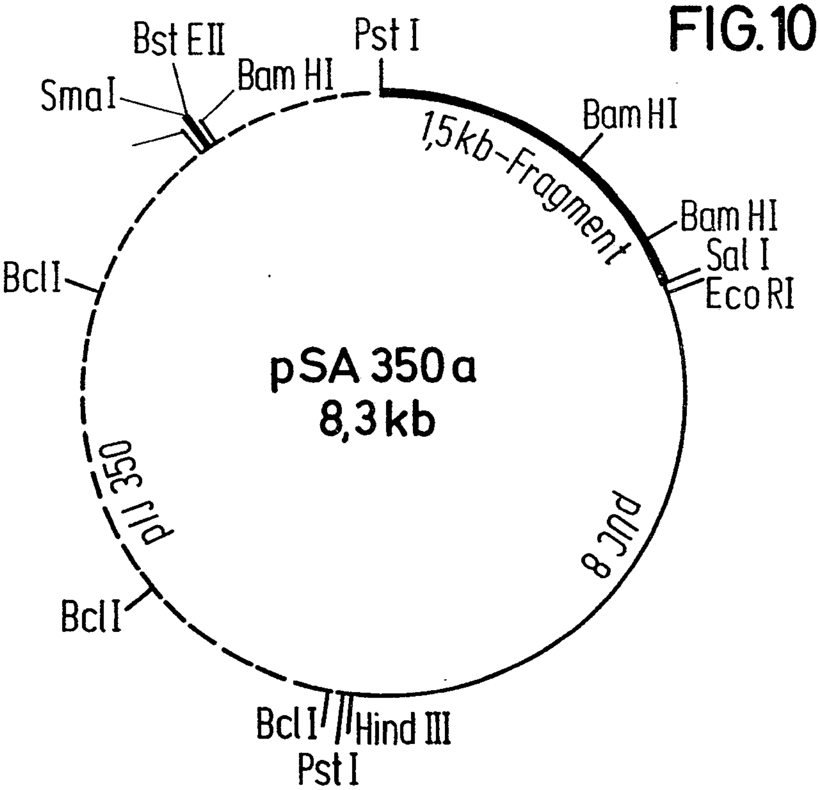

Bst EII
Pst I
Bam HI
Sma I
1,5kb-Fragment
Bam HI
Bam HI
Sal I
Eco RI
Bcl I
pSA 350 a
8,3 kb
pIJ 350
pUC 8
Bcl I
Bcl I
Hind III
Pst I